# EUROPEAN PATENT APPLICATION

(11) **EP 1 357 116 A1**
(43) Date of publication of application: **29.10.2003**
(21) Application number: 02711252.3
(22) Date of filing: 31.01.2002
(51) Int. Cl.: C07D 277/40, C07D 277/46, C07D 417/04, C07D 417/12, A61K 31/454, A61K 31/427, A61K 31/5377, A61K 31/496, A61K 31/4439, A61K 31/55, A61K 31/4545, A61P 7/00, A61P 7/04

(54) **2-ACYLAMINOTHIAZOLE DERIVATIVE OR ITS SALT**

(30) Priority: 02.02.2001 JP 2001026955
(71) Applicant: YAMANOUCHI PHARMACEUTICAL CO. LTD., Tokyo 103-8411 (JP)
(72) Inventor: KOSHIO, Hiroyuki, c/o Yamanouchi Pharm. Co., Ltd., Tsukuba-shi, Ibaraki 305-8585 (JP); KIMIZUKA, Tetsuya, c/o Yamanouchi Pharm. Co., Ltd., Itabashi-ku, Tokyo 174-8612 (JP); SUGASAWA, Keizo, c/o Yamanouchi Pharm. Co., Ltd., Tsukuba-shi, Ibaraki 305-8585 (JP); WATANUKI, Susumu, c/o Yamanouchi Pharm. Co., Ltd., Tsukuba-shi, Ibaraki 305-8585 (JP); KOGA, Yuji, c/o Yamanouchi Pharm. Co., Ltd., Tsukuba-shi, Ibaraki 305-8585 (JP); NAGATA, Hiroshi, c/o Yamanouchi Pharm. Co.,Ltd., Tsukuba-shi, Ibaraki 305-8585 (JP); SUZUKI, Ken-ichi, c/o Yamanouchi Pharm. Co., Ltd., Tsukuba-shi, Ibaraki 305-8585 (JP); ABE, Masaki, c/o Yamanouchi Pharm. Co., Ltd., Tsukuba-shi, Ibaraki 305-8585 (JP)
(74) Representative: Bates, Philip Ian
(86) International application number: JP0200755
(87) International publication number: WO02062775

(57) **Abstract**

A 2-acylaminothiazole derivative or a pharmaceutically acceptable salt thereof having a superior platelet increasing activity based on a megakaryocyte colony forming action. A compound or its salt useful for therapy of thrombocytopenia.

## Description

### TECHNICAL FIELD

The present invention relates to a novel 2-acylaminothiazole derivative or a salt thereof useful as a drug, especially a therapeutic agent for thrombocytopenia and to a pharmaceutical composition comprising the compound as an active ingredient.

### BACKGROUND ART

A platelet is a non-nucleated blood cell functioning mainly for physiological hemostasis and pathologic thrombus formation and is always produced from a megakaryocyte as a progenitor cell in a living body. A platelet originates in a pluripotent stem cell likewise other blood cells, and the pluripotent stem cell becomes a megakaryocytic progenitor cell, from which are successively produced a megakaryoblast, a promegakaryocyte and a megakaryocyte. In the step of maturation of the megakaryocyte, an immature megakaryocyte undergoes only DNA synthesis without being accompanied with cell division and becomes a polyploid. Thereafter, a cytoplasm starts maturation to form a platelet isolation membrane and causes rupture, whereby the platelet is released.

On the other hand, thrombocytopenic states such as aplastic anemia, myelodysplastic syndromes, various hematopoietic disorders in the chemotherapy or radiotherapy of malignant tumor serious symptoms such as induction of bleeding tendency. For the purpose of treating these symptoms, there have been made attempts to develop various technologies for increasing the platelet. Although powerful means for the therapy of thrombocytopenia is platelet transfusion; at present a sufficient amount of the platelet is not supplied. Further, for the reason that the life of the transfused platelet is short, it is difficult to sufficiently improve the thrombocytopenia. For this reason, development of drugs that relieve the suppresive state of hematopoietic function induced by various symptoms or treatments and promote the recovery of the platelet number is demanded.

And, it was reported that thrombopoietin (hereafter referred to as "TPO") that is a major factor contributing to differentiation of a pluripotent stem cell into a megakaryocytic cell is subjected to cloning and stimulates the differentiation and proliferation of the megakaryocytic cell to promote the production of platelet (Kaushansky, K., et al., Nature, 369, 568-571, 1994). TPO has already undergone clinical trials as a platelet increasing agent, and its usefulness and tolerability in human beings are being confirmed. However, in-clinical trials of PEG-rHuMGDF (in which the 163rd amino acid from the N-terminal of TPO is modified with polyethylene glycol) that is one kind of TPO, a neutralizing antibody was developed (Vadhan-Raj, S., *Semin Hematol.,* 37 (suppl. 4), 28-34, 2000). Accordingly, TPO is worried about immunogenicity. Further, since TPO is a protein, it is decomposed within digestive tracts and hence, it is not practically useful as a drug for oral administration. For the same reason, it is also considered that a low-molecular peptide is not practically useful as a drug for oral administration. Under such circumstances, for the purpose of therapy for thrombocytopenia, development of non-peptide platelet increasing agents that are less in immunogenicity and can be orally administered proceeds.

As compounds having the foregoing platelet increasing activity, there are known benzodiazepine derivatives (JP-A-11-152276), pyrrolophenanthridine derivatives (JP-A-10-212289), pyrrolophthalimide derivatives (JP-A-2000-44562), acylhydrazone derivatives (WO 99/11262), diazonaphthalene derivatives (WO 2000/35446), and pyrrolocarbazole derivatives (WO 98/09967).

Further, WO 01/07423 describes that compounds represented by the following general formula (A) have a platelet increasing activity. 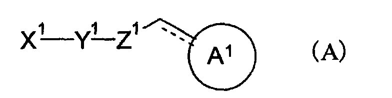 (In the formula, the symbols are as defined in the patent document.)

This patent document describes compounds in which X¹ represents an optionally substituted thiazole, and Y¹ represents -NHCO-. However, in the general formula (A) of this patent document, the substituent of Z¹ is limited to a substituent having A¹, such as a thiazolyl group. Further, in this patent document, compounds having a nitrogen atom substituted at the 5-position of the thiazole are not specifically described by working examples and others.

Moreover, WO 01/53267 describes that compounds represented by the following general formula (B) have a platelet increasing activity.

X¹―Y¹―Z¹―W¹ (B)

(In the formula, the symbols are as defined in the patent document.)

This patent document describes compounds in which X¹ represents an optionally substituted thiazole, and Y¹ represents -NHCO-. However, in the general formula (B) of this patent document, the substituent of Z¹ is limited to W¹. Further, in this patent document, compounds having a nitrogen atom substituted at the 5-position of the thiazole are not specifically described by working examples and others.

In addition, 2-acylamino-5-thiazole derivatives in which only an indole ring is bound to an amide bond at the 2-position of the thiazole are disclosed as a chlolecystokinin or gastrin receptor antagonist in Japanese Patent No. 3,199,451; and 2-disubstituted amino-4-arylthiazol-5-ylalkanoic acids are disclosed as a compound having an anti-inflammatory characteristic in *Chemical and Pharmaceutical Bulletin*, Vol. 25, No. 9, pp. 2292-2299. However, any of these documents do not mention at all the platelet increasing activity.

Under the foregoing circumstances, for the purpose of therapy for thrombocytopenia, development of non-peptide platelet increasing agents that are less in immunogenicity and can be orally administered is eagerly demanded.

### DISCLOSURE OF THE INVENTION

The present inventors made extensive and intensive investigations with respect to compounds having a platelet increasing activity. As a result, it has been found that a novel 2-acylaminothiazole derivative has a superior platelet increasing activity, leading to accomplishment of the invention.

The compound of the invention is a 2-acylaminothiazole derivative structurally characterized in that an acylamino group is substituted at the 2-position thereof and that a nitrogen atom of a nitrogen-containing heterocycle is directly bound to the 5-position thereof. Further, the compound of the invention is pharmacologically characterized by having a platelet increasing activity based on a megakaryocyte colony formation promoting action.

Specifically, according to the invention, there is provided a 2-acylaminothiazole derivative represented by the following general formula (I) or a pharmaceutically acceptable salt thereof, which is useful as a therapeutic agent for thrombocytopenia.

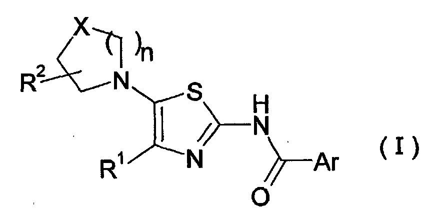

In the formula, the symbols have the following meanings:

Ar represents phenyl or pyridyl, each of which may be substituted with one or more groups selected from the group consisting of lower alkyl, -CO-lower alkyl, -COO-lower-alkyl, -OH, -O-lower alkyl, -OCO-lower alkyl, and halogen;
R¹ represents aryl or pyridyl, each of which may be substituted with one or more groups selected from the group consisting of lower alkyl, -CO-lower alkyl, -COO-lower alkyl, -OH, -O-lower alkyl, -OCO-lower alkyl, and halogen;
R² represents a group selected from the group consisting of -H, -OH, -COOH, -COO-lower alkyl, carbamoyl which may be substituted with one or two lower alkyls, amino which may be substituted with one or two lower alkyls, and cyclic amino, provided that one or more of this group may be present on the ring;
-X- represents -CH₂-, -O-, -S-, or -N(R³)-;
R³ represents optionally substituted lower alkyl, cycloalkyl, optionally substituted aryl, optionally substituted aryl-lower alkyl, optionally substituted heteroaryl, optionally substituted heteroaryl-lower alkyl, -CO-lower alkyl, -COO-lower alkyl, or carbamoyl which may be substituted one or two lower alkyls; and
n represents an integer of from 1 to 3.

Compounds represented by the foregoing general formula (I), wherein X represents -N(R³)-, and n is 2 or 3, or pharmaceutically acceptable salts thereof are preferable. Compounds represented by the foregoing general formula (I), wherein X represents -N(R³)-, n is 2 or 3, and Ar represents phenyl or pyridyl, each of which may be substituted with one or more groups selected from the group consisting of -OH, -O-lower alkyl, and -OCO-lower alkyl, or pharmaceutically acceptable salts thereof are more preferable. Particularly preferred examples include:
3,5-dimethoxy-N-(5-morpholin-4-yl-4-phenylthiazol-2-yl)benzamide,
N-[5-(4-cyclohexylpiperazin-1-yl)-4-(4-fluorophenyl)thiazol-2-yl]-2-m ethoxyisonicotinamide,
3-chloro-N-[5-(4-cyclohexylpiperazin-1-yl)-4-(4-fluorophenyl)thiazol-2-yl]-4-hydroxybenzamide,
3,5-dimethoxy-N-(5-piperidin-1-yl-4-pyridin-4-ylthiazol-2-yl)benzam ide, or
4-{[5-(4-cyclohexylpiperazin-1-yl)-4-phenylthiazol-2-yl]carbamoyl}ph enyl acetate, or
pharmaceutically acceptable salts thereof.

Further, according to the invention, there is provided a pharmaceutical composition comprising, as an active ingredient, a compound represented by the foregoing general formula (I); a compound represented by the foregoing general formula (I), wherein X represents -N(R³)-, and n is 2 or 3; a compound represented by the foregoing general formula (I), wherein X represents -N(R³)-, n is 2 or 3, and Ar represents phenyl or pyridyl, each of which may be substituted with one or more groups selected from the group consisting of -OH, -O-lower alkyl, and -OCO-lower alkyl; or a pharmaceutically acceptable salt thereof. Concretely, the foregoing pharmaceutical composition is a pharmaceutical composition as a megakaryocyte colony forming promoter, a pharmaceutical composition as a platelet increasing agent, or a pharmaceutical composition as a therapeutic agent for thrombocytopenia.

The compounds of the invention will be further described below.

In this description, the term "lower alkyl" means a linear or branched carbon chain having from 1 to 6 carbon atoms (C₁₋₆), and specific examples include methyl, ethyl, propyl, isopropyl, butyl, tert-butyl, pentyl, neopentyl, and hexyl, etc. Of these are preferable C₁₋₃ alkyls including methyl, ethyl, propyl and isopropyl. Examples of the substituent acceptable in the "optionally substituted lower alkyl" for R³ include -O-lower alkyl and -O-aryl, etc.

The term "aryl" means an aromatic ring comprising carbon atoms and is preferably a monocyclic to tricyclic aromatic ring having from 6 to 14 carbon atoms (C₆₋₁₄). Specific examples include phenyl and naphthyl, with phenyl being preferred. Examples of the substituent acceptable in the "optionally substituted aryl" and "optionally substituted aryl-lower alkyl" for R³ include lower alkyl, -O-lower alkyl, halogen, nitro, and cyano, etc.

The term "heteroaryl" means a monovalent group of a monocyclic to tricyclic aromatic ring having one or more hetero atom such as nitrogen, oxygen, and sulfur, and specific examples include pyridyl, pyrazyl, pyridazyl, pyrrolyl, imidazolyl, thienyl, furanyl, thiazolyl, oxazolyl, indolyl, quinolyl, and benzothiazolyl, etc. Examples of the substituent acceptable in the "optionally substituted heteroaryl" and "optionally substituted heteroaryl-lower alkyl" for R³ include lower alkyl, -O-lower alkyl, halogen, nitro, and cyano, etc.

The term "cycloalkyl" means a 3- to 8-membered carbon ring, and specific examples include cyclopropyl, cyclobutyl, cyclohexyl, and cyclooctyl, etc.

The term "cyclic amino" means a monovalent group of 3- to 10-membered cyclic amine, and preferably a 5 to 7-membered cyclic amine, which may contain nitrogen, oxygen or sulfur. Specific examples include pyrrolidino, piperidino, azepinyl, N-methylpiperazino, N-methylhomopiperazino, morpholino, and thiomorpholino, etc.

Examples of the "halogen" include a fluorine, a chlorine, a bromine, and an iodine atom.

In this description, the term "one or more" that is used for defining the number of groups preferably means "from one to three", and more preferably "one or two".

The compound of the invention represented by the general formula (I) may possibly have an asymmetric carbon atom depending on the kind of the substituent(s), and optical isomers may be present based on this. The invention includes all of mixtures or isolated compounds of these optical isomers. Further, in the compound of the invention, there may be possibly present tautomeric isomers. The invention includes isolated compounds or mixtures of these isomers.

Further, the compounds of the invention may possibly form an acid-addition salt. The invention includes such a salt so far as it is a pharmaceutically acceptable salt. Specifically, examples include acid addition salts of an inorganic acid such as hydrochloric acid, hydrobromic acid, hydroiodic acid, sulfuric acid, nitric acid, and phosphoric acid; and acid addition salts of an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonic acid, aspartic acid, and glutamic acid. In addition, the invention includes various-hydrates, solvates and crystal polymorphisms of the compound of the invention and its pharmaceutically acceptable salt. Incidentally, the compound of the invention includes all of so-called prodrugs, i.e., compounds that will be metabolized and converted into the compound of the foregoing general formula (I) or its salt within a living body. As the group to form the prodrug are enumerated those groups described in *Prog. Med*., 5, 2157-2161 (1985) and *Iyakuhin No Kaihatsu* (Development of Drugs), Vol. 7, "Molecular Design", 163-198 (1990), by Hirokawa Publishing Co.

### Production process:

The compound of the invention and its pharmaceutically acceptable salt can be produced through application of various known synthesis processes by utilizing the characteristic based on the basic skeleton thereof or kinds of the substituents. Representative production processes will be enumerated below. Incidentally, in some case, it is effective on the production technology that depending on the kind of a functional group, the functional group is replaced by a protective group, i.e., a group that can be readily converted into the functional group in a state of the starting material or intermediates. Thereafter, if desired, the protective group is removed, thereby enabling to obtain the desired compound. Examples of such a functional group include a hydroxyl group, a carboxyl group and an amino group. Examples of the protective group thereof include the protective groups as described in Greene and Wuts, *Protective Groups in Organic Synthesis (third edition),* and these may be properly used depending on the reaction condition.

### (First production process)

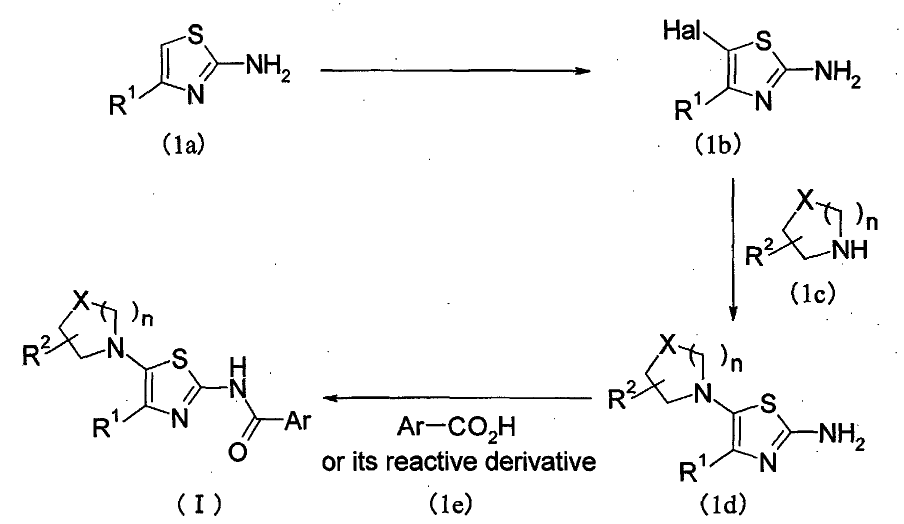

(In the formula, R¹, R², Ar, X and n have the same meanings as defined above, and Hal represents halogen, hereafter the same.)

This production process is a process in which an optionally protected substituted aromatic carboxylic acid represented by the formula (1e) or its reactive derivative and an optionally protected 2-aminothiazole derivative represented by the formula (1d or its salt are subjected-to amidation in a customary manner, and the protective group(s) is removed, if desired, to produce the compound (I) of the invention.

Examples of the reactive derivative of the compound (1e) include usual esters such as methyl esters, ethyl esters, and tert-butyl esters; acid halides such as acid chlorides and acid bromides; acid azides; active esters with N-hydroxybenzotriazole, p-nitrophenol, N-hydroxysuccimide, etc.; symmetric acid anhydrides; and mixed acid anhydrides with an alkyl carbonate, p-toluenesufonic acid, etc.

Further, when the compound (1e) is reacted in a liberated acid or reacted without isolating the active ester or acid halide, it is suitable to use a condensing agent such as dicyclohexylcarbodiimide, carbonyldiimidazole, diphenylphosphoryl azide, diethylphosphoryl cyanide, and 1-ethyl-3-(3-dimethylaminopropyl)carbodiimide hydrochloride.

The reaction varies depending on the reactive derivative and the condensing agent to be used but is usually carried out in an organic solvent that is inert to the reaction, such as halogenated hydrocarbons inclusive of dichloromethane, dichloroethane, chloroform, and carbon tetrachloride, aromatic hydrocarbons inclusive of benzene, toluene, xylene, ethers inclusive of diethyl ether, tetrahydrofuran, and dioxane, esters inclusive of ethyl acetate (EtOAc), acetonitrile, N,N-dimethylformamide (DMF), and dimethyl sulfoxide (DMSO), under cooling, or at a temperature of from cooling temperature to room temperature, or at a temperature of from room temperature to an elevated temperature.

Incidentally, in some case, it is advantageous for-making the reaction proceed smoothly that the reaction is carried out by using an excessive amount of the compound (1e) or in the presence of a base such as N-methylmorpholine, trimethylamine, triethylamine, N,N-dimethylaniline, pyridine, 4-(N,N-dimethylamino)pyridine, picoline, and lutidine. Further, a salt comprising a weak base and a strong acid, such as pyridine hydrochloride, pyridine p-toluenesulfonate, and N,N-dimethylaniline hydrochloride, may be used. Pyridine may be used as the solvent.

Especially, it is suitable to carry out the reaction in a solvent such as acetonitrile and DMF in the presence of a base such as pyridine and N,N-dimethylaniline, or by using pyridine as a solvent.

The starting compound (1d) that is used for this reaction can be produced by halogenating the 5-position of a thiazole derivative represented by the formula (1a) to synthesize a compound (1b) to which is then exerted a cyclic amine (1c), as shown in the foregoing reaction scheme. If desired, the protective group(s) can be removed at an arbitrary stage. Incidentally, the compound (1b may be used for the next reaction without being isolated.

As the halogenating agent that is used in the halogenation step, any halogenating agents that are usually used for halogen substitution reaction of hydrogen on an aromatic ring will do. Suitable examples include single halogen such as chlorine and bromine; and perbromides of pyridine, α-pyrrolidone, quaternary ammonium, dioxane, etc., such as dioxane dibromide, phenyltrimethylammonium tribromide, pyridinium hydrobromide perbromide, and pyrrolidone hydrotribromide. Further, imide-based halogenating agents such as N-bromosuccinimide and N-chlorosuccinimide; hydrogen halides such as hydrogen chloride and hydrogen-bromide; and metal reagents such as copper(II) halides such as copper(II) bromide and copper(II) chloride, can also be used.

In the case where the single halogen or perbromide is used as the halogenating agent, the halogenating agent may be exerted to the compound (1a) in an organic solvent that is inert to the reaction, such as halogenated hydrocarbons; ethers; alcohols such as methanol (MeOH), ethanol (EtOH), 2-propanol (iPrOH), and ethylene glycol; aromatic hydrocarbons; acetic acid; and esters. At this time, if desired, the reaction may be carried out in the presence of a small amount of a catalyst such as a hydrogen halide, and the reaction temperature is preferably from -30°C to the refluxing temperature of the solvent to be used.

In the case where the hydrogen halide is used as the halogenating agent, the halogenating agent can be exerted to the compound (1a) in an acidic solution or a basic solution (such as a sodium hydroxide solution) of the hydrogen halide. At this time, the reaction temperature is preferably from -30°C to the refluxing temperature of the solvent to be used. Further, it is usually advantageous that the reaction using the metal reagent is carried out by dissolving the compound (1a) in an organic solvent that is inert to the reaction, such as halogenated hydrocarbons, ethers, alcohols, aromatic hydrocarbons, acetic acid, and esters, or water, or a mixed solvent thereof and exerting the metal reagent to the solution in the optional presence of a small amount of a catalyst such as a hydrogen halide at room temperature or upon heating.

The cyclic amine (1c) is exerted to the thus obtained compound (1b in an organic solvent that is inert to the reaction, such as aprotic-polar solvent inclusive of DMF, N-methyl-2-pyrrolidone, and DMSO, halogenated hydrocarbons, ethers, and aromatic hydrocarbons, or water, or a mixed solvent thereof, to synthesize the compound (1d). At this time, the reaction temperature is preferably from room temperature to the refluxing temperature of the solvent to be used.

Incidentally, in some case, it is advantageous for making the reaction proceed smoothly that the reaction is carried out by using an excessive amount of the cyclic amine (1c) or in the presence of a base such as N-methylmorpholine, triethylamine, diethylisopropylamine, N,N-dimethylaniline, pyridine, 4-(N,N-dimethylamino)pyridine, picoline, and lutidine.

### (Second production process)

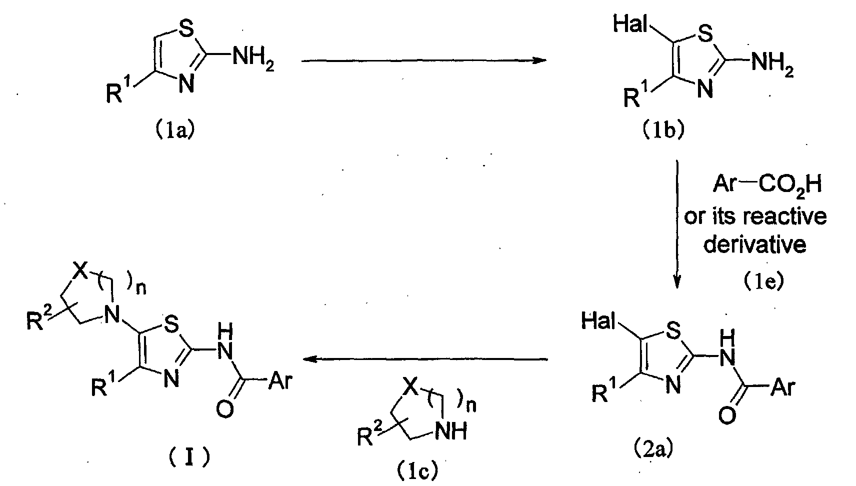

This production process is a process in which the compound (1b as synthesized in the method shown in the first production process and an optionally protected aromatic carboxylic acid represented by the formula (1e) or its reactive-derivative are subjected to amidation in a customary manner to synthesize an optionally protected 5-halo-2-aminothiazole derivative represented by the formula (2a), to which is then exerted the cyclic amine (1c), and if desired, the protective group(s) is removed, to produce the compound (I) of the invention.

In any of the steps, the method as shown in the first production process can be applied.

The thus produced compound of the invention is isolated and purified in a liberated state or as a salt after subjecting to salt formation in a customary manner. The isolation and purification are carried out by applying a usual chemical operation such as extraction, concentration, distillation, crystallization, filtration, recrystallization, and various kinds of chromatography.

Various isomers can be isolated in a customary manner by utilizing a difference in physicochemical properties among the isomers. For example, in the case of racemic mixtures, the racemic compound can be introduced into an optically pure isomer by a general racemic resolution method such as a method in which the racemic compound is introduced into a diastereomer salt with a general optically active acid such as tartaric acid, which is then subjected to optical resolution. Further, the mixture of diastereomers can be separated by fractional crystallization or various kinds of chromatography. Moreover, it is possible to produce optically active compounds by using a proper optically active starting material.

### INDUSTRIAL APPLICABILITY

The compound of the invention and its salt have a superior platelet increasing activity.

Accordingly, the compound of the invention is useful for therapy and/or prevention of various kinds of thrombocytopenia, such as aplastic anemia, thrombocytopenia in myelodysplastic syndromes , thrombocytopenia by chemotherapy or radiotherapy of malignant tumor, idiopathic thrombocytopenic purpura , thrombocytopenia in liver diseases , and thrombocytopenia by HIV. Further, in the case where there is possibility to cause a reduction of platelet by chemotherapy or radiotherapy, the compound of the invention may be previously administered before undergoing the treatment.

The pharmacological actions of the compound of the invention were confirmed by the following assay.

### <Megakaryocyte colony formation promoting action>

A human CD34⁺ cell was cultured at 37°C for 10 to 14 days in the presence of a test compound in a 2-well chamber slide by using MegaCult™-C (produced by StemCell Technologies). According to the manufacturer's instructions, the slide was dehydrated, fixed, and then stained with an anti-glycoprotein IIb/IIIa antibody. A colony containing three or more of the glycoprotein IIb/IIIa positive megakaryocyte cells was defined as one colony, and the number of colonies per well was microscopically measured. The assay was replicated thrice, and an average value was evaluated as the number of megakaryocyte colonies.

**(Table 1)**

| Megakaryocyte colony formation promoting action of the compound of the invention | | | |
|---|---|---|---|
| Concentration of test compound (Example 2) (µM) | 0.3 | 1.0 | 3.0 |
| Number of megakaryocyte colonies | 5.2 | 19.0 | 34.8 |

Thus, it has been confirmed that the compound of the invention has a superior megakaryocyte colony formation promoting action and has a platelet increasing activity based on this action.

Further, it has been confirmed that the compound of the invention has a human platelet increasing activity in human hematopoietic stem cells engrafted mice, previously verified to produce human platelets.

The drug of the invention can be prepared by a usually employed method using one or two or more of the compound of the invention represented by the general formula (I) and a pharmaceutical carrier, excipient and other additives as used for formulation. The administration may be in any form of oral administration by tablets, pills, capsules, granules, powders, liquids, etc., or parenteral administration by injections such as intravenous or intramuscular injection, suppositories, transnasal administration, transmucous administration, dermal administration, etc.

As a solid composition for the oral administration according to the invention, tablets, powders, or granules are used. In such a solid composition, one or two or more active substances are mixed with at least one inert diluent such as lactose, mannitol, glucose, hydroxypropyl cellulose, microcrystalline cellulose, starch, polyvinylpyrrolidone, and magnesium metasilicate aluminate. The composition may contain additives other than the inert diluent, such as a lubricant such as magnesium stearate, a disintegrating agent such as cellulose calcium glycolate, a stabilizer such as lactose, and a dissolution aid such as glutamic acid and aspartic acid, according to the customary method. If desired, the tablets or pills may be coated by a sugar coating such as sugar, gelatin, hydroxypropyl cellulose, and hydroxypropylmethyl cellulose phthalate, or a film made of a gastric-soluble or intestinal soluble substance.

The liquid composition for oral administration contains a pharmaceutically acceptable emulsion agent, solution agent, suspending agent, syrup, or elixir and contains a generally employed inert diluent such as purified water and ethanol. In addition to the inert diluent, this composition may contain an auxiliary agent such as a wetting agent and a suspending agent, a sweetener, a flavor, an aromatic, or an antiseptic.

The injection for parenteral administration contains a sterile aqueous or non-aqueous solution agent, suspending agent or emulsion agent. Examples of the aqueous solution agent or suspending agent include distilled water or physiological saline for injection. Examples of the non-aqueous solution agent or suspending agent include propylene glycol, polyethylene glycol, vegetable oils such as olive oil, alcohols, and Polysolvate 80. Such a composition may also contain an auxiliary agent such as an antiseptic, a wetting agent, an emulsifier, a dispersing agent, a stabilizer such as lactose, and a dissolution aid such as glutamic acid and aspartic acid. These compositions are sterilized by, for example, filtration through a bacteria-holding filter, compounding with an anti-bacterial agent, or irradiation. Further, these can be used by producing a sterile solid composition and dissolving it in sterile water or a sterile solvent for injection before the use.

Usually, in the case of the oral administration, it is proper that the dose of the drug per day is from about 0.0001 to 50 mg per kg, preferably from about 0.001 to 10 mg per kg, and more preferably from 0.01 to 1 mg per kg of the body weight and that the drug is administered once or dividedly two to four times. In the case of the intravenous administration, it is proper that the dose of the drug per day is from about 0.0001 to 1 mg per kg, and preferably from about 0.0001 to 0.1 mg per kg of the body weight and that the drug is administered once or dividedly several times. The dose is properly determined depending on the individuals while taking into consideration the symptom, age and sex.

### BEST MODE FOR CARRYING OUT THE INVENTION

The invention will be specifically described below with reference to the following Examples, but it should not be limited thereto. Incidentally, among the starting compounds to be used in the following Examples, novel substances are included, and the production processes of such starting materials from known compounds will be described with reference to the Referential Examples.

### Referential Example 1

To a solution of 3.0 mL of 3-fluoroacetophenone in 80 mL of THF was added 9.19 g of phenyltrimethylammonium tribromide, and the mixture was stirred at room temperature for one hour. An insoluble matter was removed by filtration, and the solvent was distilled off in vacuo. To a solution of the resulting residue in 50 mL of iPrOH was added 3.72 g of thiourea, and the mixture was stirred upon heating at 80°C for 2 hours. The reaction mixture was evaporated off, to which was then added a saturated sodium hydrogencarbonate solution, and the mixture was extracted with chloroform. The organic layer was rinsed with-saturated salt water and dried over magnesium sulfate, and the solvent was distilled off to obtain 3.93 g of 2-amino-4-(3-fluorophenyl)thiazole.
FAB-MS(M+H)⁺: 195

### Referential Example 2

To a solution of 1 g of 2-amino-4-phenylthiazole in 20 mL of chloroform was added dropwise a solution of 0.29 mL of bromine in 2 mL of chloroform, and the mixture was stirred at room temperature for 30 minutes. The reaction mixture was distilled off, to which was then added 10 mL of morpholine, and the mixture was stirred at 120°C for 75 minutes. The reaction mixture was distilled off in vacuo, to which was then added chloroform, and the organic layer was rinsed with a saturated sodium hydrogencarbonate aqueous solution and saturated salt water and then dried over sodium sulfate. The solvent was distilled off in vacuo, and the resulting residue was subjected to silica gel column chromatography and eluted with hexane-EtOAc (2:1). There was thus obtained 625 mg of 2-amino-5-morpholin-4-yl-4-phenylthiazole.

Compounds of Referential Examples 3 to 54 as shown in Tables 2 to 5 were produced in the same manner as in Referential Example 2 while using the respective corresponding starting materials.

Incidentally, the abbreviations shown below are used in the table (hereinafter the same).
- Rex:: Referential Example No.
- R:: Substituent in the general formula (Me: methyl, Et: ethyl, nPr: normal propyl, Ph: phenyl)
- Date:: Physicochemical data (NMR:¹H-NMR δ (ppm), MS:
FAB-MS(M+H)⁺)

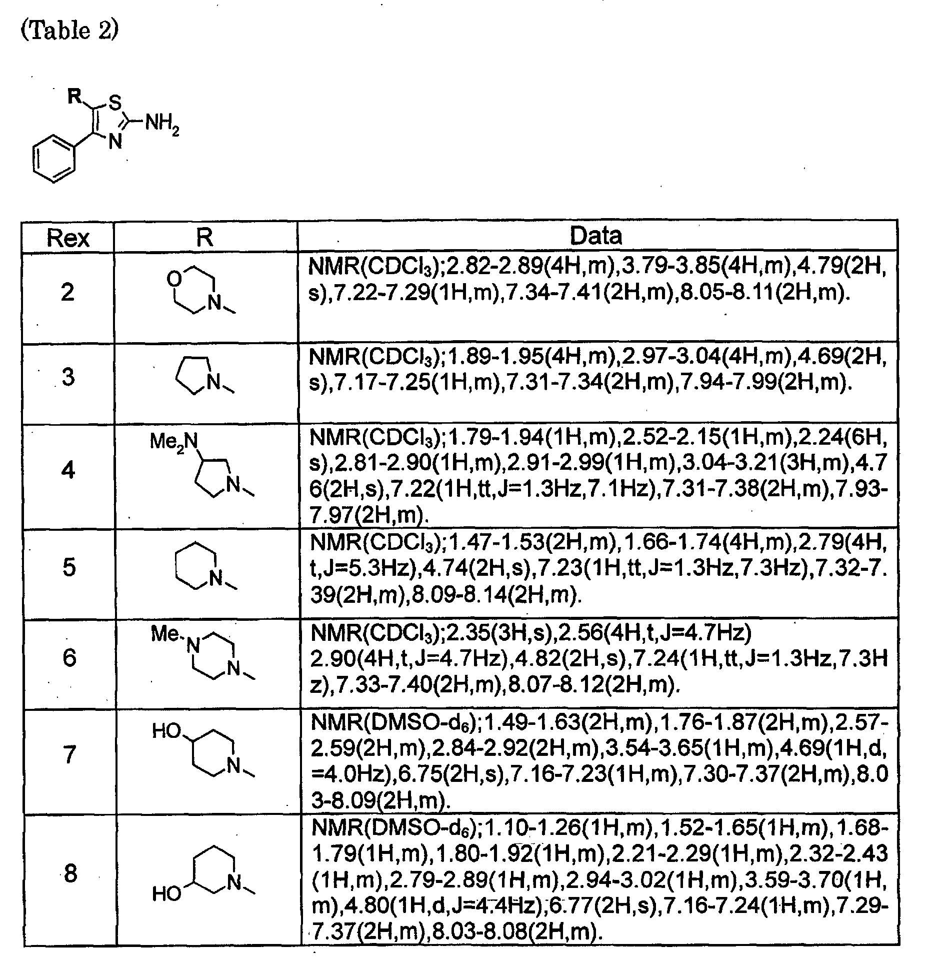

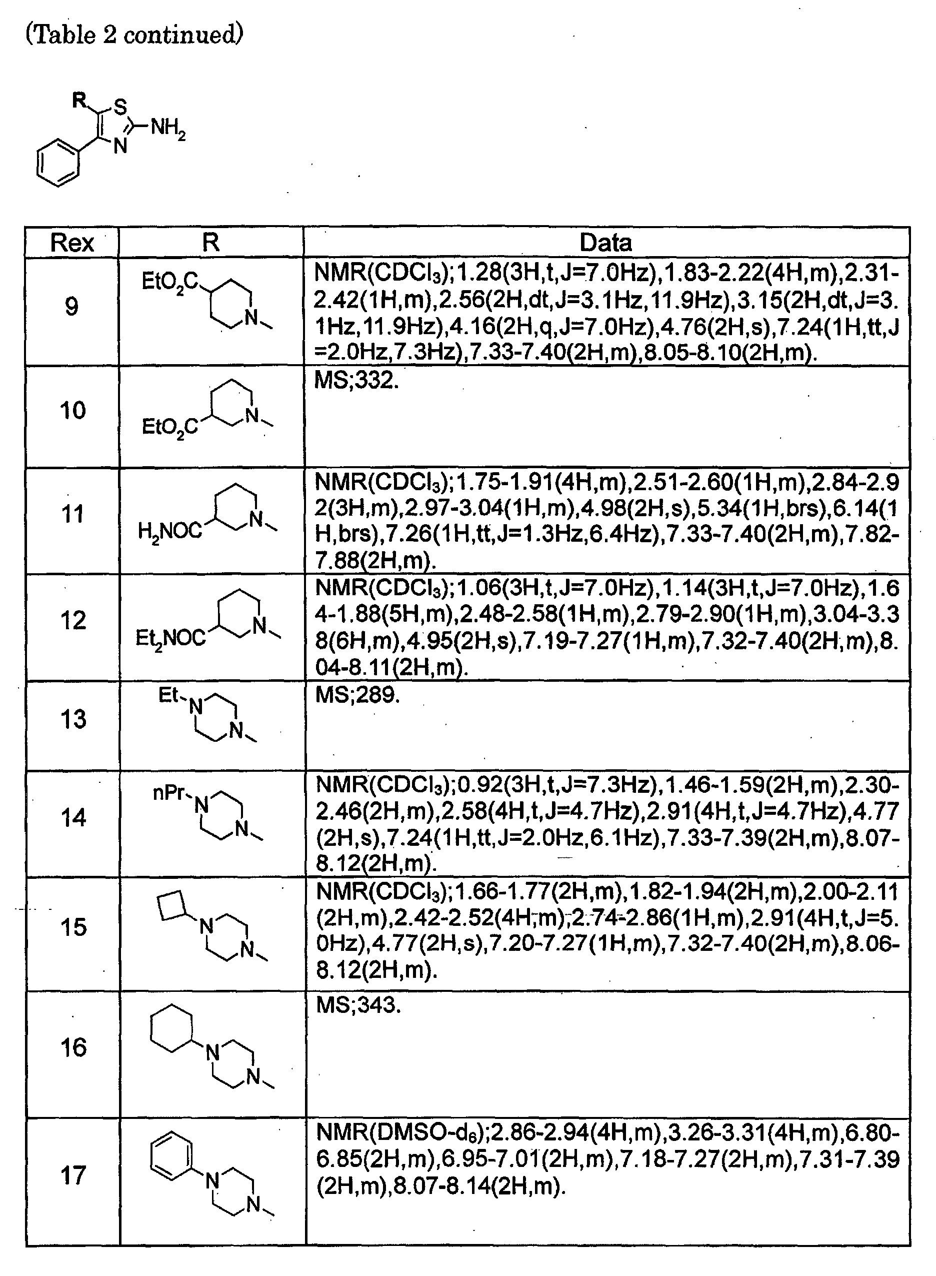

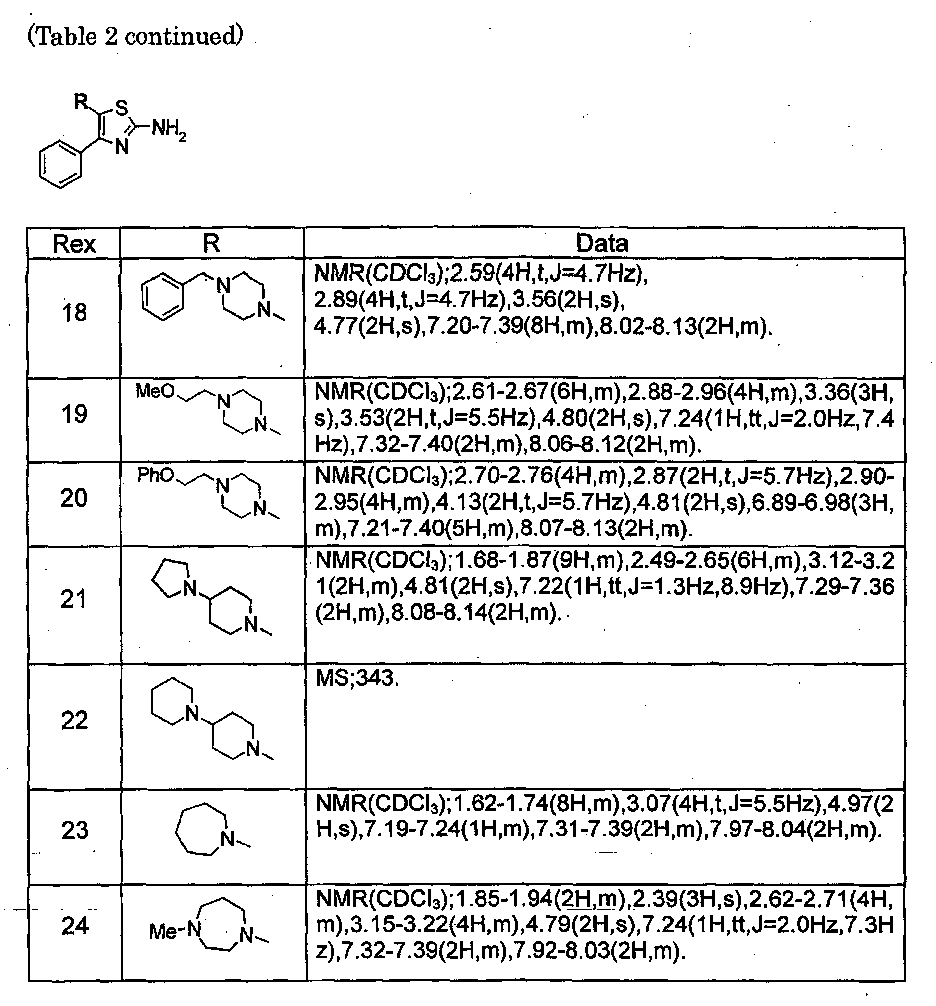

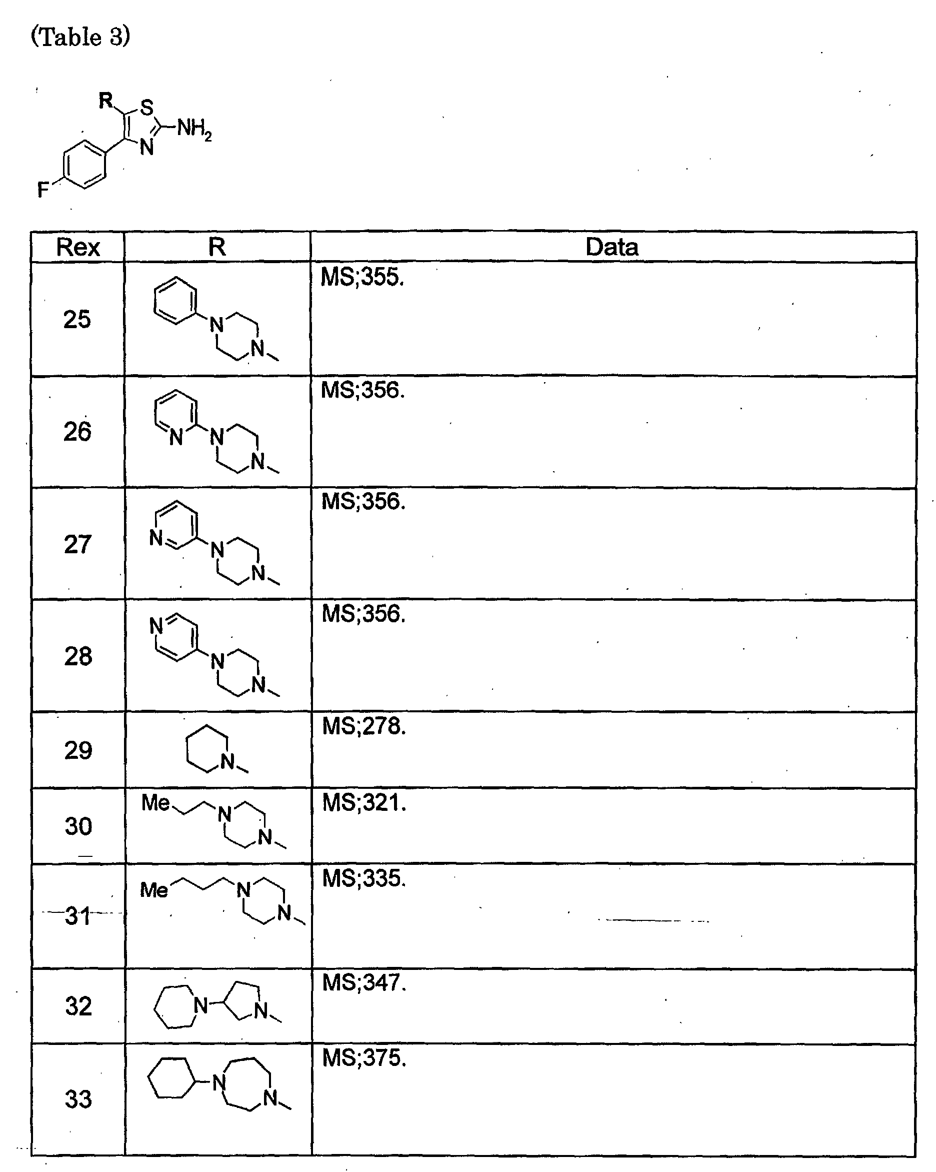

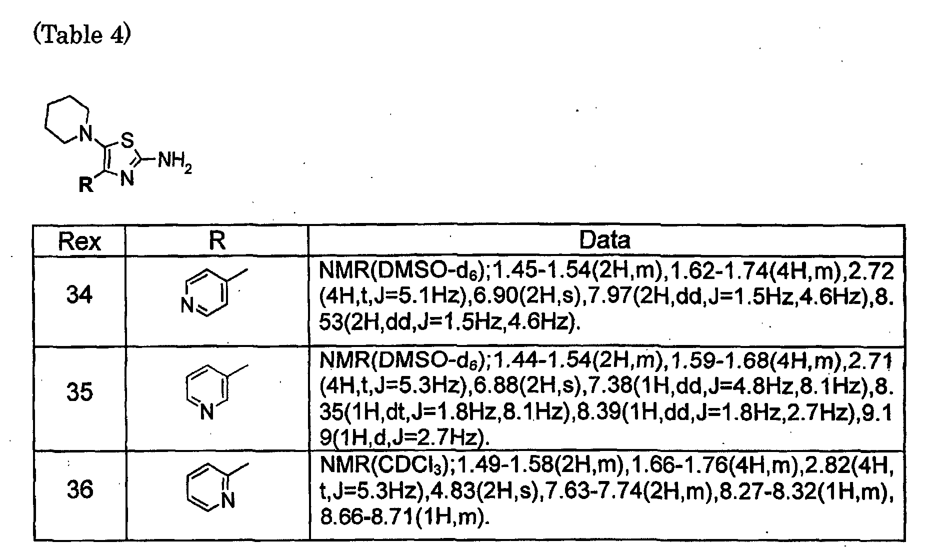

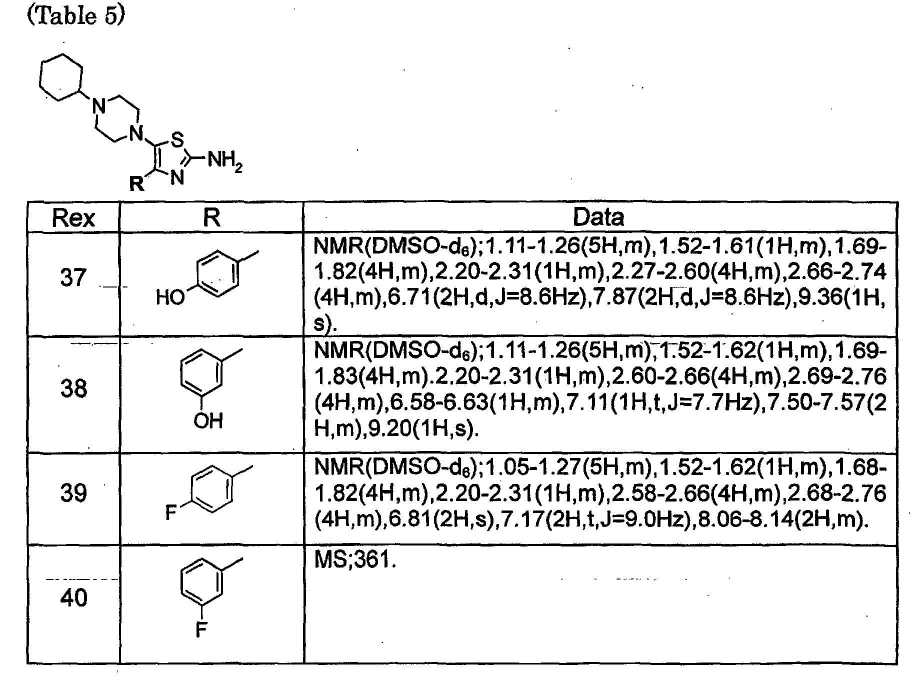

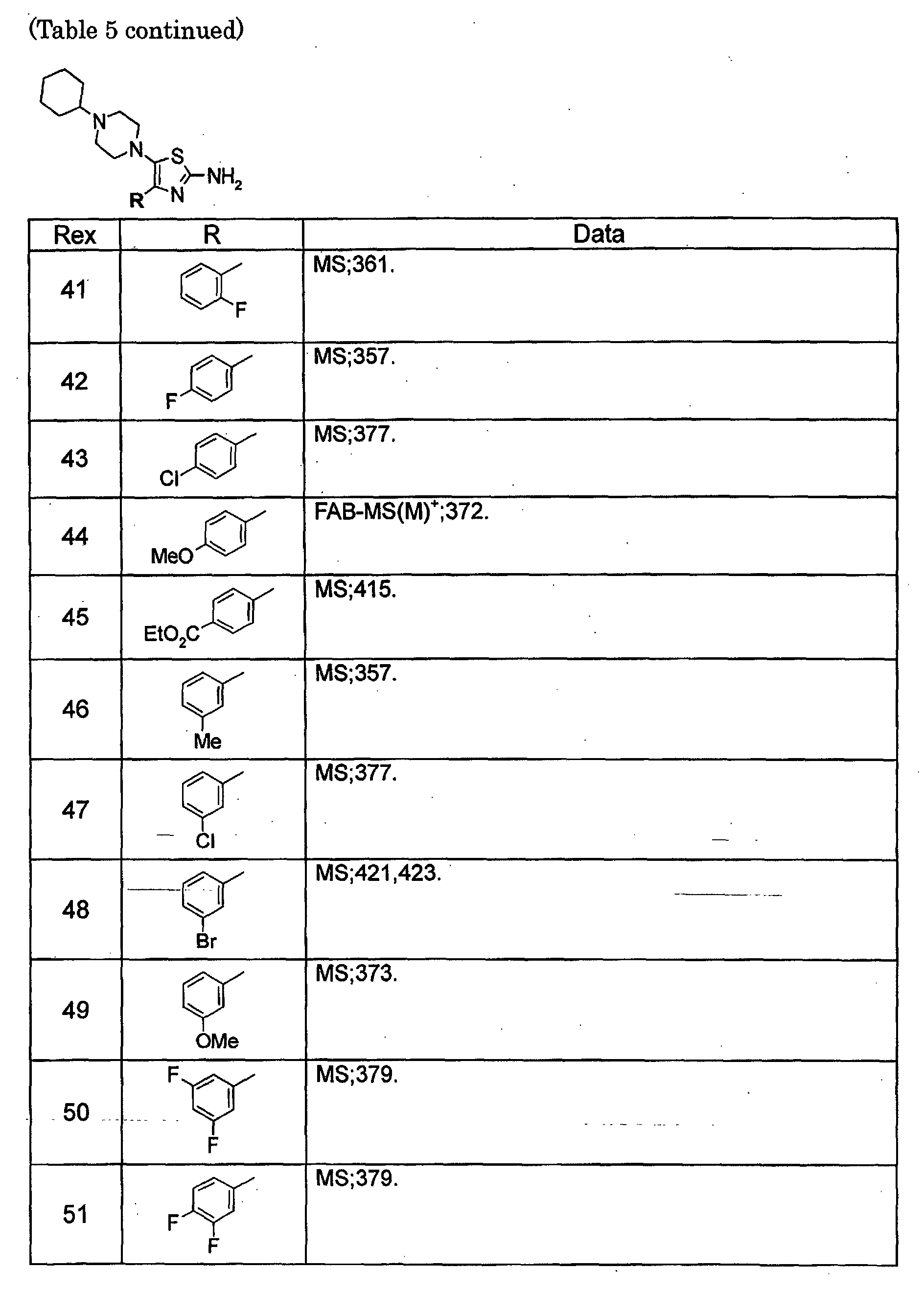

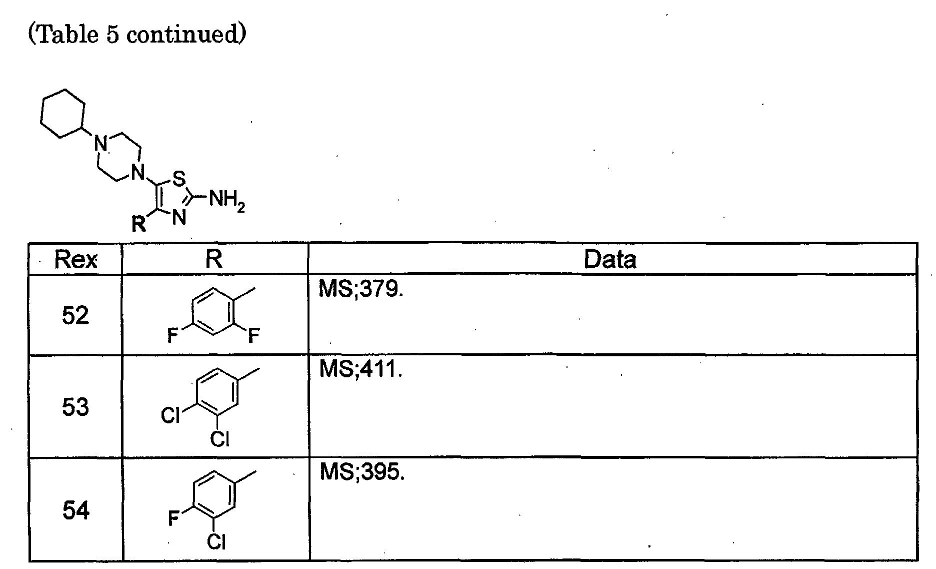

### Example 1

To a solution of 570 mg of 3,5-dimethoxybenzoic acid in 10 mL of THF were added a catalytic amount of DMF and 0.4 mL of thionyl chloride under ice cooling, and after elevating the temperature to room temperature, the mixture was stirred for 3 hours. The reaction mixture was distilled off, and a solution of 620 mg of the compound of Referential Example 2 in 10 mL of pyridine was added dropwise to the resulting residue under ice cooling. After elevating the temperature to room temperature, the mixture was stirred for 16 hours. The reaction mixture was distilled off in vacuo, to which was then added chloroform, and the organic layer was rinsed with a saturated sodium hydrogencarbonate aqueous solution and saturated salt water and then dried over sodium sulfate. The solvent was distilled off in vacuo, and the resulting residue was subjected to silica gel column chromatography and eluted with hexane-EtOAc (3:1). The eluate was distilled off in vacuo, and the resulting residue was dissolved in 10 mL of EtOAc, to which was then added 0.65 mL of a 4M hydrochloric acid-EtOAc solution. The mixture was stirred for a while, and the solvent was then distilled off in vacuo. The resulting residue was recrystallized from acetonitrile to obtain 641 mg of 3, 5-dimethoxy-N-(5-morpholin-4-yl-4-phenylthiazol-2-yl)benzamide hydrochloride.
¹H-NMR (DMSO-*d*₆): δ 2.87-2.95 (4H, m), 3.75-3.78 (4H, m), 3.83 (6H, s), 6.74 (1H, t J=1.9 Hz), 7.28-7.32 (3H, m), 7.44 (2H, t, J=7.8 Hz), 8.13-8.18 (2H, m), 12.50 (1H, brs)
FAB-MS(M+H)⁺: 425
Melting point (°C): 169-172

### Example 2

To a solution of 1.60 g of the compound of Referential Example 39 in 30 mL of THF were added 680 mg of 2-methoxyisonicotinic acid and 1.02 g of WSC-HCl and the mixture was stirred at room temperature for 4 days. To the reaction mixture was added a saturated sodium hydrogencarbonate aqueous solution, and the mixture was extracted with chloroform. The organic layer was rinsed with saturated salt water and then dried over sodium sulfate. The solvent was distilled off, and the residue was purified by silica gel column chromatography (eluting solution: hexane-EtOAc = 5:1 to 2:1). The elutant was dissolved in a mixed solvent of 20 mL of EtOAc and 25 mL of EtOH, to which was then added 2.22 mL of a 4M hydrochloric acid-EtOAc solution, and the mixture was stirred for a while. A deposit was collected by filtration to obtain 822 mg of N-[5-(4-cyclohexylpiperazin-1-yl)-4-(4-fluorophenyl)thiazol-2-yl]-2-methoxyi sonicotinamide.
¹H-NMR (DMSO-*d*₆): δ 1.09-1.18 (1H, m), 1.21-1.36 (2H, m), 1.41-1.54 (2H, m), 1.58-1.69 (1H, m), 1.80-1.89 (2H, m), 2.14-2.25 (2H, m), 3.16-3.40 (7H, m), 3.48-3.57 (2H, m), 3.93 (3H, s), 7.28 (2H, t, J=9.3 Hz), 7.45 (1H, s), 7.56 (1H, dd, J=1.4Hz, 5.4Hz), 8.12-8.19 (2H, m), 8.37 (1H, d, J=5.4 Hz), 11.15 (1H, brs), 12.86 (1H, brs)
FAB-MS(M+H)⁺: 496
Melting point (°C): 263-266

### Example 3

To a solution of 360 mg of the compound of Referential Example 39 in 10 mL of DMF were added 345 mg of 3-chloro-4-hydroxybenzoic acid, 383 mg of WSC·HCl, 270 mg of 1-hydroxybenzotriazole, and 244 mg of 4-(dimethylamino)pyridine. After elevating the temperature from room temperature to 90°C, the mixture was stirred for 4 days. The reaction mixture was distilled off, to which was then added water, and the mixture was extracted with EtOAc. The organic layer was rinsed with water and saturated salt water and then dried over sodium sulfate. The solvent was distilled off, and the residue was purified by silica gel column chromatography (eluting solution: hexane-EtOAc = 5:1 to 1:1). The elutant was dissolved in 15 mL of EtOAc, to which was then added 1.6 mL of a 0.1M hydrochloric acid-EtOAc solution, and the mixture was stirred for a while. A deposit was collected by filtration to obtain 57 mg of 3-chloro-N-[5-(4-cyclohexylpiperazin- 1 -yl)-4-(4-fluorophenyl)thiazol-2-yl]-4-hydroxybenzamide hydrochloride.
¹H-NMR (DMSO-*d*₆):δ 1.09-1.50 (5H, m), 1.59-1.69 (1H, m), 1.81-1.91 (2H, m), 2.09-2.19 (2H, m), 3.08-3.44 (7H, m), 3.52- 3.60 (2H, m)_{,} 7.09 (1H, d, J=8.3 Hz), 7.27 (2H, t, J=8.8 Hz), 7.94 (1H, dd, J=8.3 Hz, 1.9 Hz), 8.11-8.21 (3H, m), 9.85 (1H, brs), 11.16 (1H, s), 12.45 (1H, s)
FAB-MS(M+H)+: 515
Melting point (°C): 270-272 (decomposed)

### Example 4

To a solution of 330 mg of the compound of Example 7 in 5 mL of EtOH and 5 mL of THF was added 0.80 mL of a 1M sodium hydroxide aqueous solution under ice cooling. After elevating the temperature to room temperature, the mixture was stirred for 8 days. To the reaction mixture were added 0.80 mL of a 1M hydrochloric acid aqueous solution and water, and the mixture was extracted with chloroform. The organic layer was rinsed with saturated salt water and then dried over magnesium sulfate. The solvent was distilled off, and the resulting residue was subjected to silica gel column chromatography and eluted with chloroform-MeOH (9:1). The eluate was concentrated in vacuo, and the resulting residue was recrystallized from EtOH to obtain 52 mg of 1-{2-[(3,5-dimethoxybenzoyl)amino]-4-phenylthiazol-5-yl}piperidine-4-carbo xylic acid.
¹H-NMR (DMSO-*d*₆):δ 1.69-1.82 (2H, m), 1.90-1.99 (2H, m), 2.37-2.46 (1H, m), 2.67-2.76 (2H, m), 3.09-3.17 (2H, m), 3.83 (6H, s), 6.73 (1H, t, J=2.0 Hz), 7.25-7.31 (3H, m), 7.44 (2H, t, J=7.3 Hz), 7.13 (2H, d, J=7.3 Hz), 12.40 (1H, brs)
FAB-MS(M+H)⁺: 468

### Example 5

To a solution of 300 mg of the compound of Example 38 in 10 mL of MeOH was added 0.71 mL of a 1M sodium hydroxide aqueous solution under ice cooling. The mixture was stirred for one hour under ice cooling, to which was then added 0.71 mL of a 1M hydrochloric acid aqueous solution. The solvent was distilled off in vacuo. To the residue were added chloroform and a saturated sodium hydrogencarbonate aqueous solution, and the organic layer was rinsed with saturated salt water and then dried over magnesium sulfate. The solvent was distilled off, and the resulting residue was subjected to silica gel column chromatography and eluted with hexane-EtOAc (1:1). The eluate was concentrated in vacuo, and the resulting residue was dissolved in 10 mL of EtOAc, to which was then added 0.41 mL of 4M hydrochloric acid-EtOAc. The mixture was stirred at room temperature for 3 hours. A deposited crystal was collected by filtration to obtain 144 mg of N-[5-(4-cyclohexylpiperazin-1-yl)-4-phenylthiazol-2-yl]-4-hydroxybenzamide dihydrochloride.
¹H-NMR (DMSO-*d*₆):δ 0.61-1.18 (1H, m), 1.21-1.33 (2H, m), 1.40-1.53 (2H, m), 1.59-1.67 (1H, m), 1.81-1.89 (2H, m), 2.15-2.23 (2H, m), 3.17-3.38 (7H, m), 3.49-3.57 (2H, m), 6.89 (2H, d, J=8.8 Hz), 7.31 (1H, t, J=7.8 Hz), 7.45 (2H, t, J=7.8 Hz), 8.01 (2H, d, J=8.8 Hz), 8.12 (2H, d, J=7.8 Hz), 11.03 (1H, brs), 12.28 (1H, brs)
FAB-MS(M+H)⁺: 463

Compounds of Examples 7 to 36 as shown in Tables 6 to 8 were produced in the same manner as in Example 1, compounds of Examples 37 to 39 as shown in Tables 9 to 10 were produced in the same manner as in Example 2 or 3, and a compound of Example 6 as shown in Table 6 was produced in the same manner as in Example 4, respectively, while using the respective corresponding starting materials.

Incidentally, the abbreviations shown below are used in the tables (hereinafter the same).
- Ex:: Example No.
- R, Ar:: Substituent in the general formula (Ac: acetyl)
- salt:: Salt (no description: free form, HCl: hydrochloride)
- mp:: Melting point (°C)

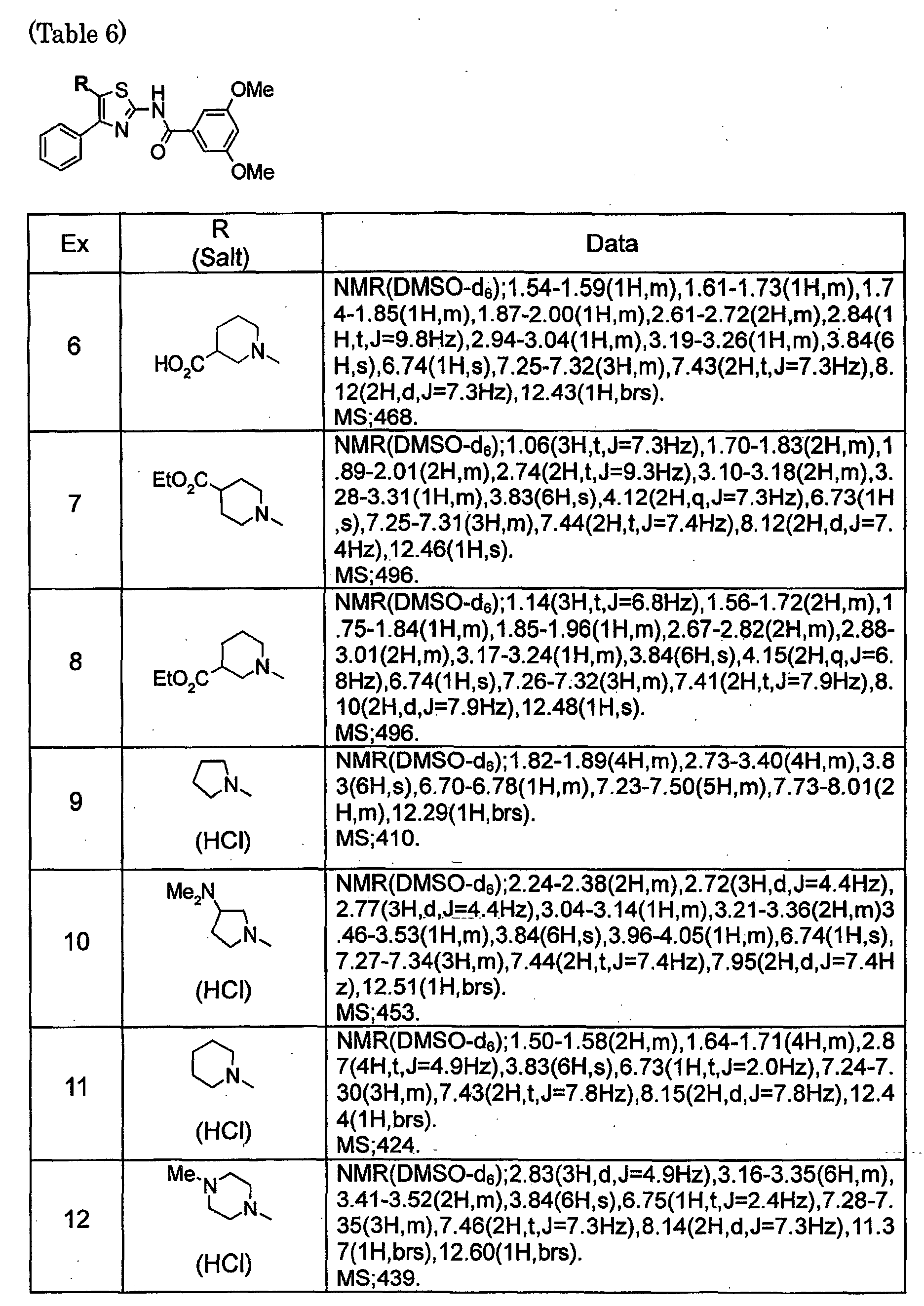

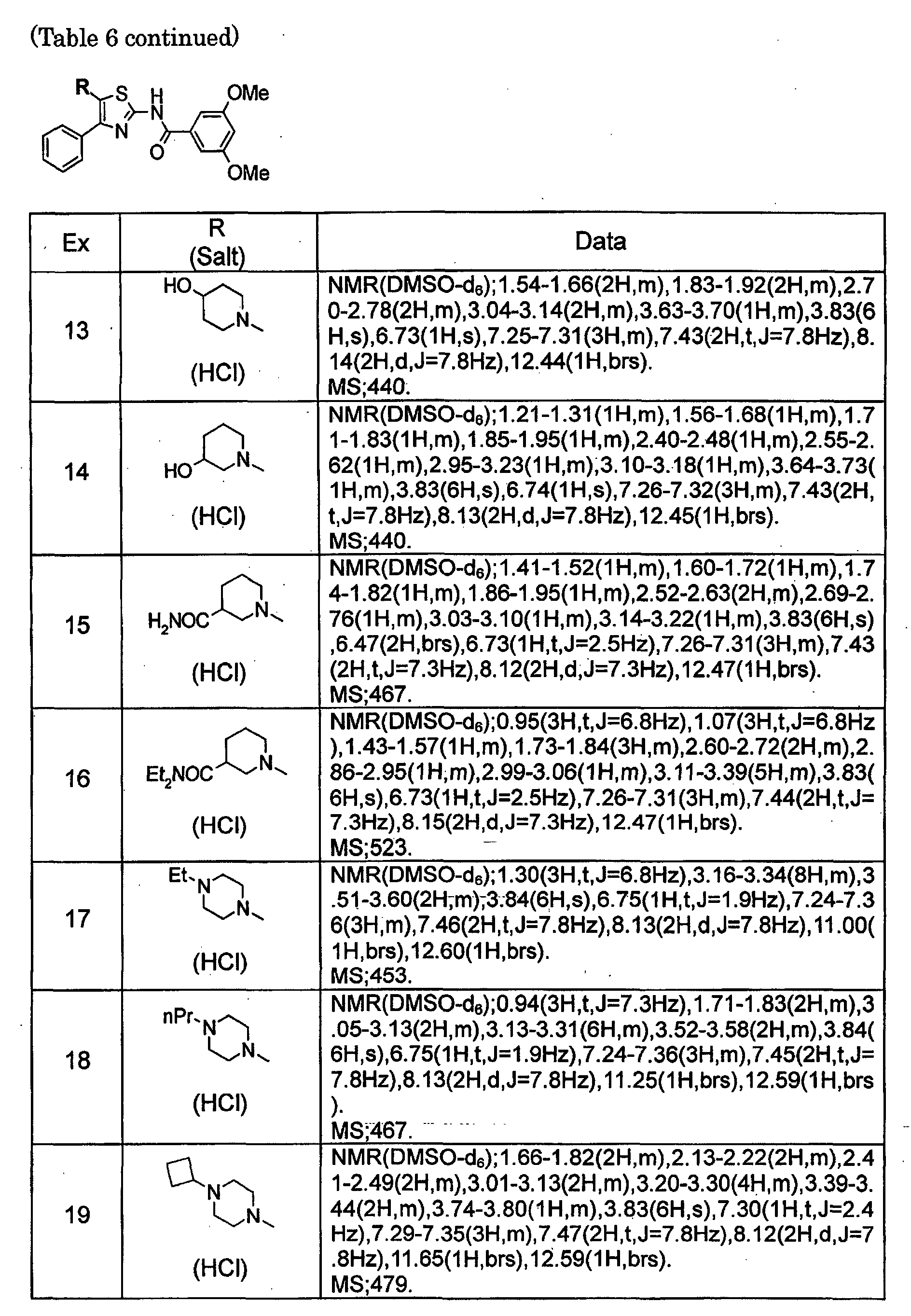

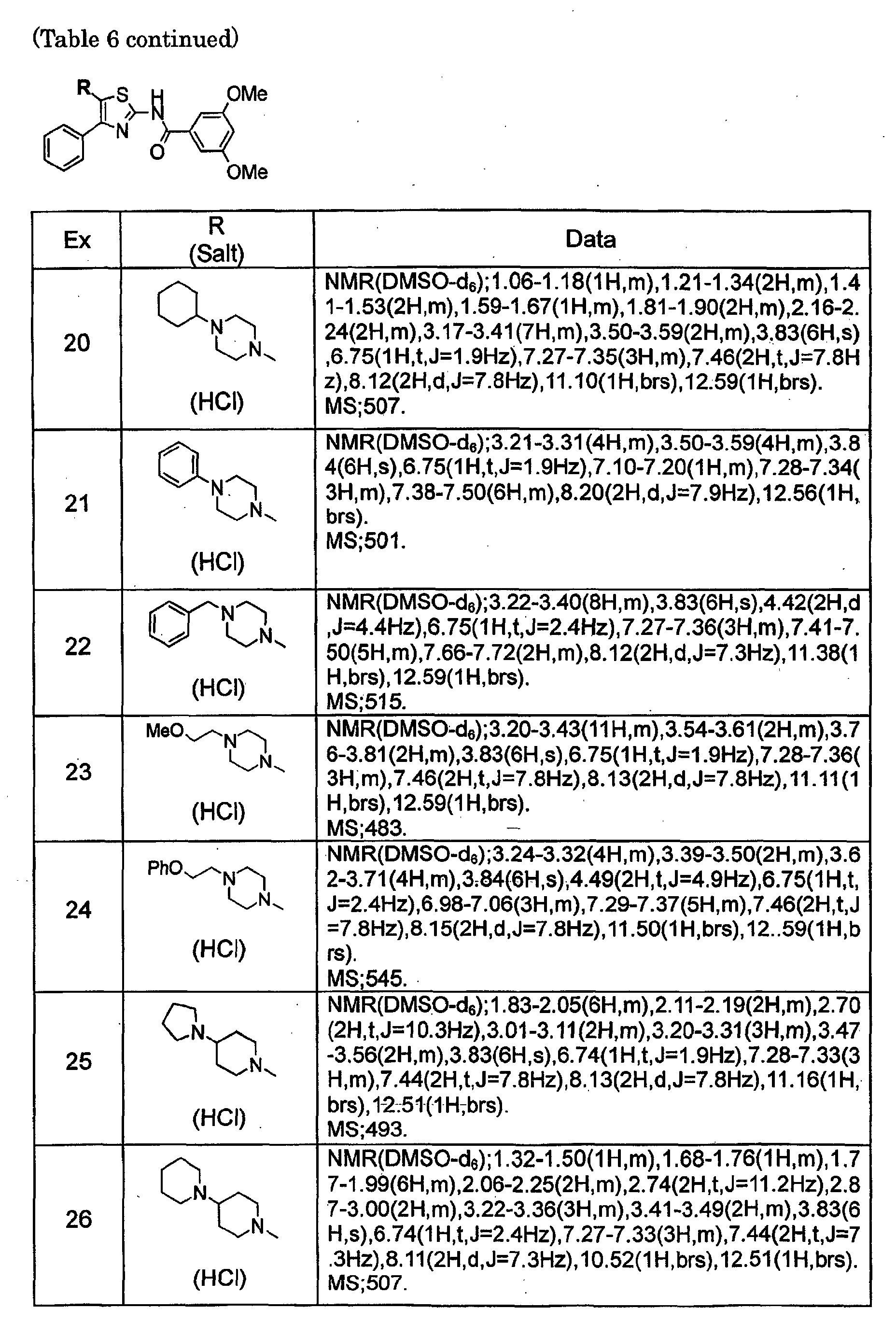

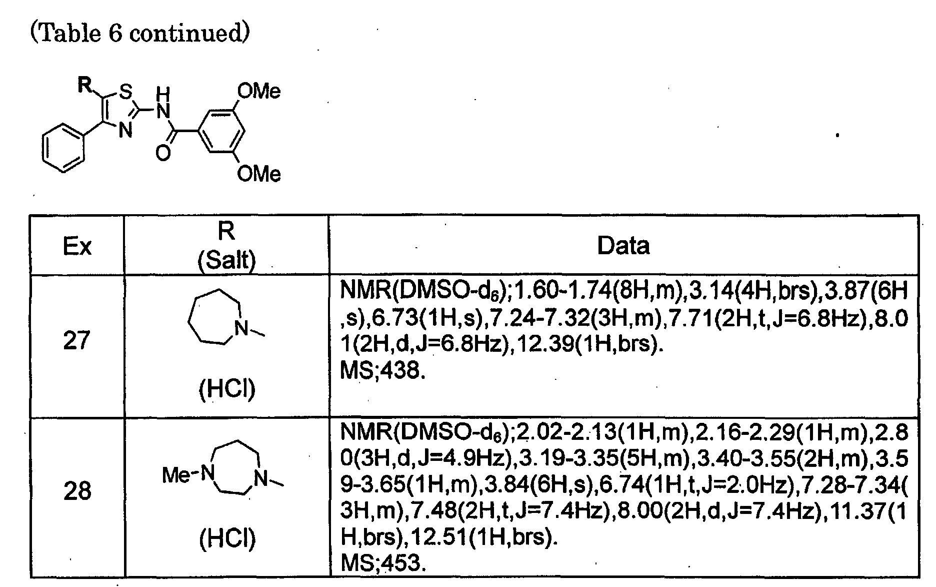

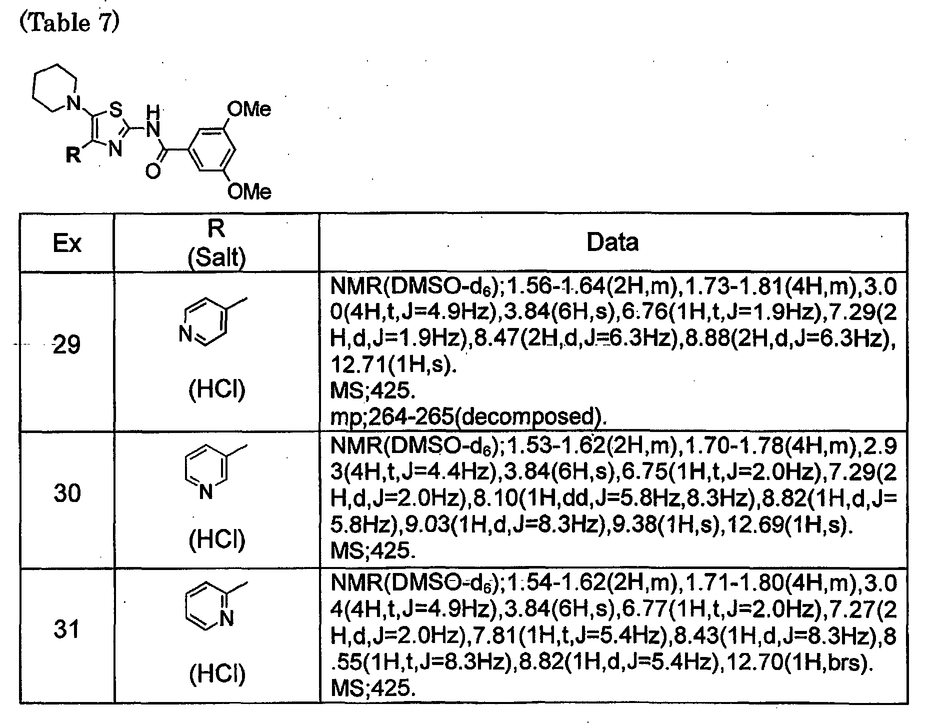

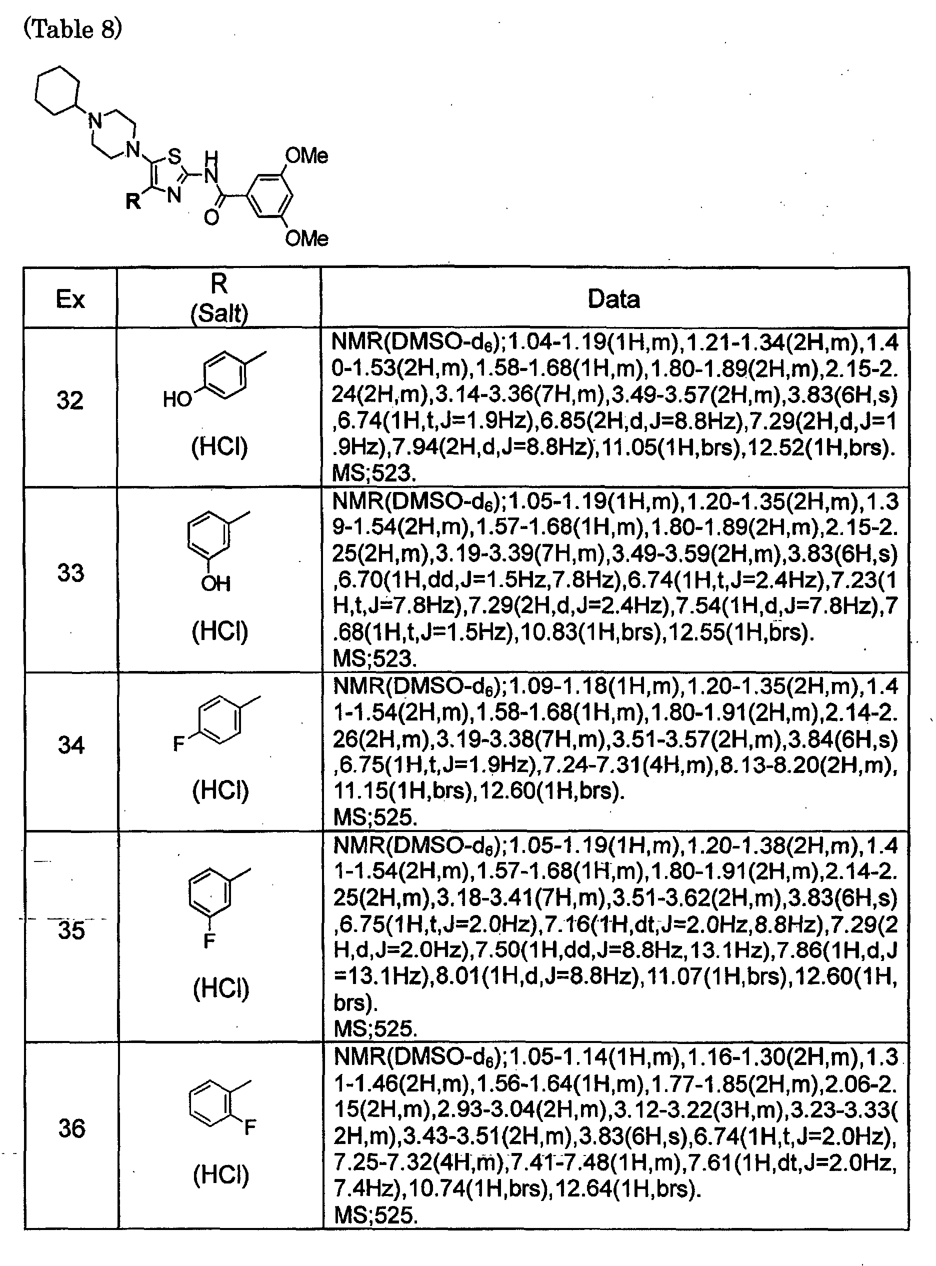

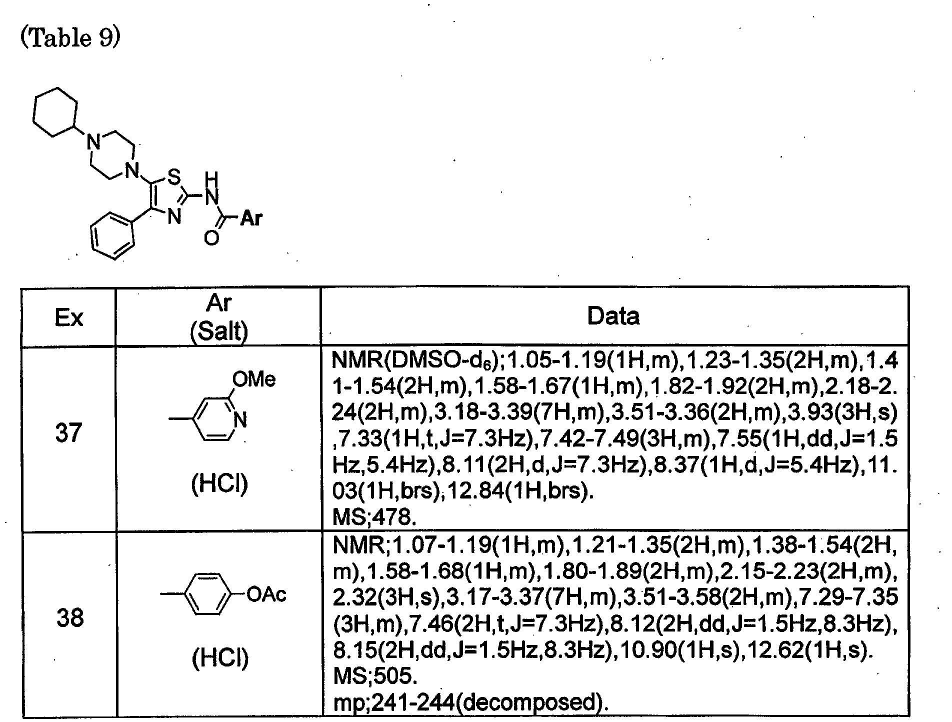

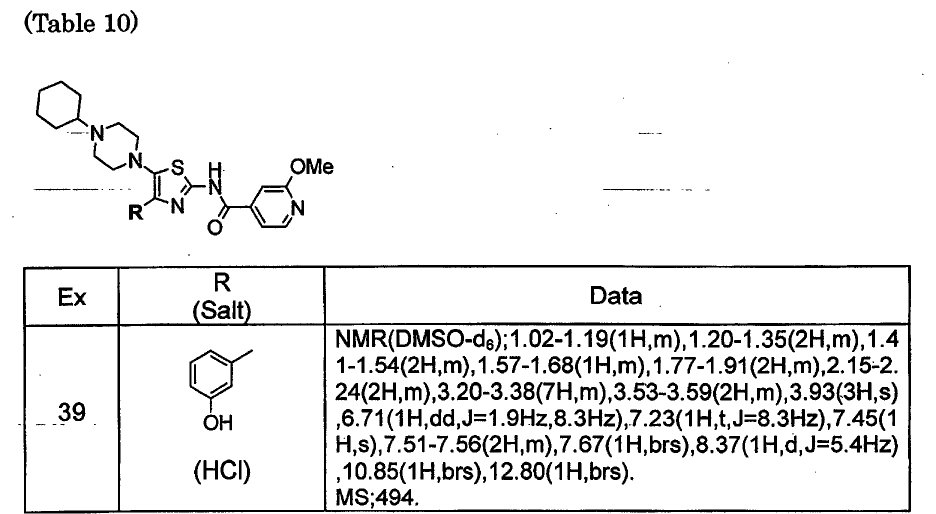

Structures of other compounds of the invention will be shown in Table 11. These compounds can be easily synthesized in the foregoing production processes or the processes as described in the Examples, or by undergoing slight modifications within the range obvious to those skilled in the art.

Incidentally, the abbreviations shown below are used in the table.
No: Compound number
R¹, R², Ar: Substituent in the general formula (cPent: cyclopentyl, cHex: cyclohexyl, cHep: cycloheptyl, nBu: normal butyl, nHex: normal hexyl, iPr: isopropyl, tBu: tertiary butyl, Mor: morpholin-4-yl, pipa: piperazinyl, pipe: piperidinyl; Py: pyridyl, di: di. Incidentally, the numeral before the substituent means the substitution position. Accordingly, 2-MeO-4-Py, 3,5-diMeO-Ph and 4-cHex-1-pipa represent 2-methoxypyridin-4-yl, 3,5-dimethoxyphenyl and 4-cyclohexylpiperazin-1-yl, respectively.)

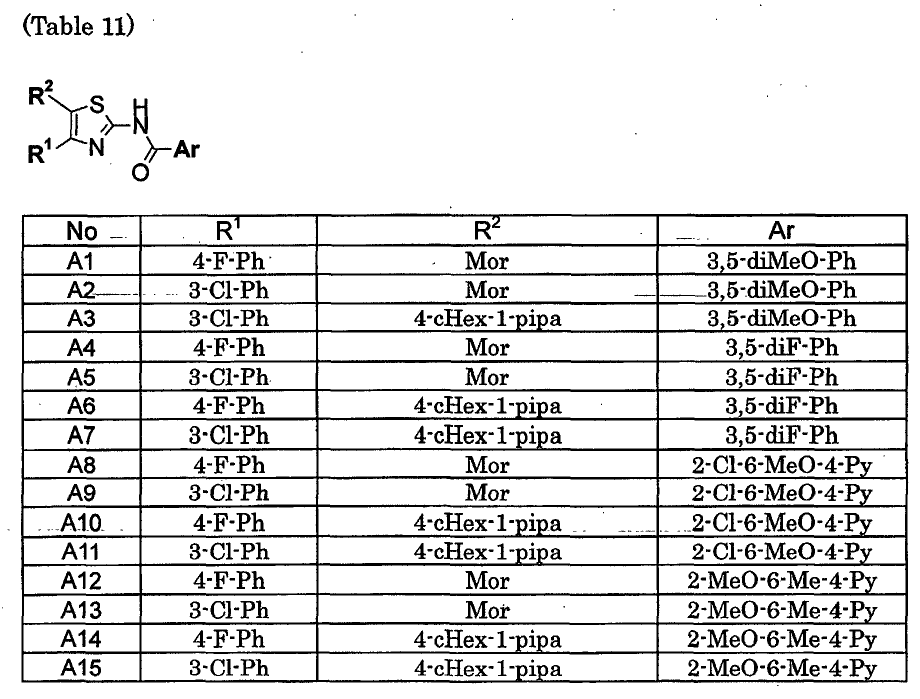

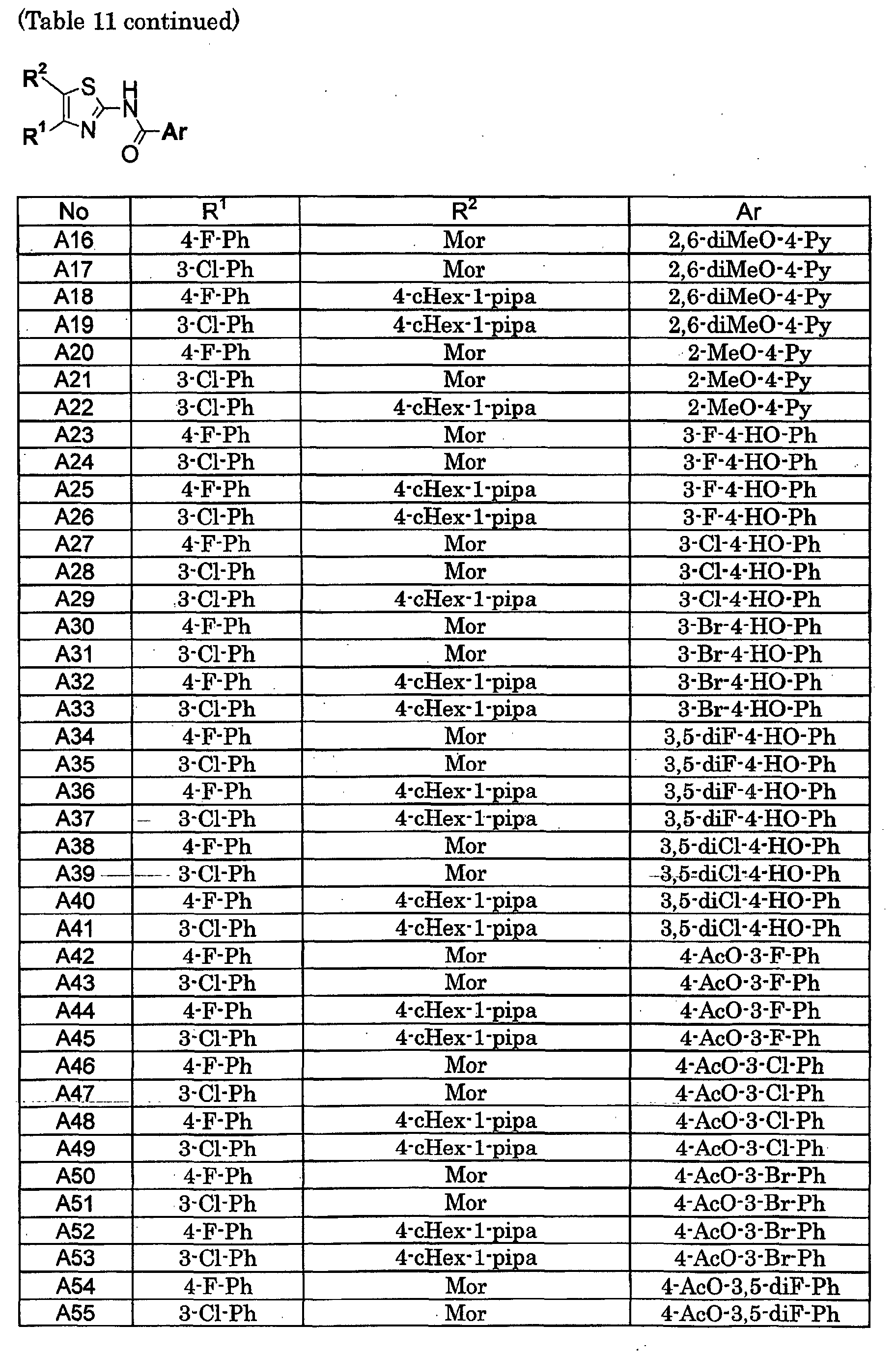

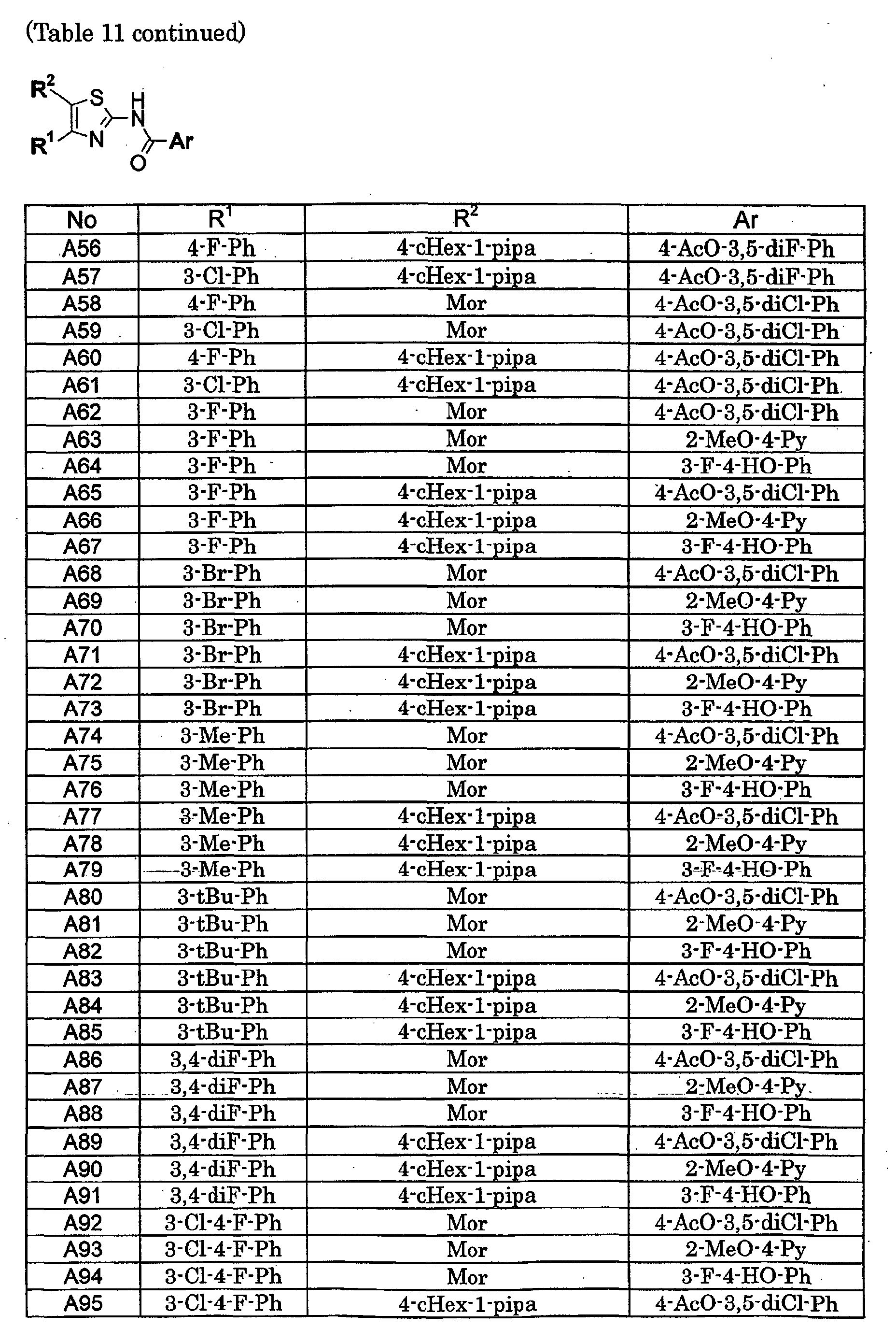

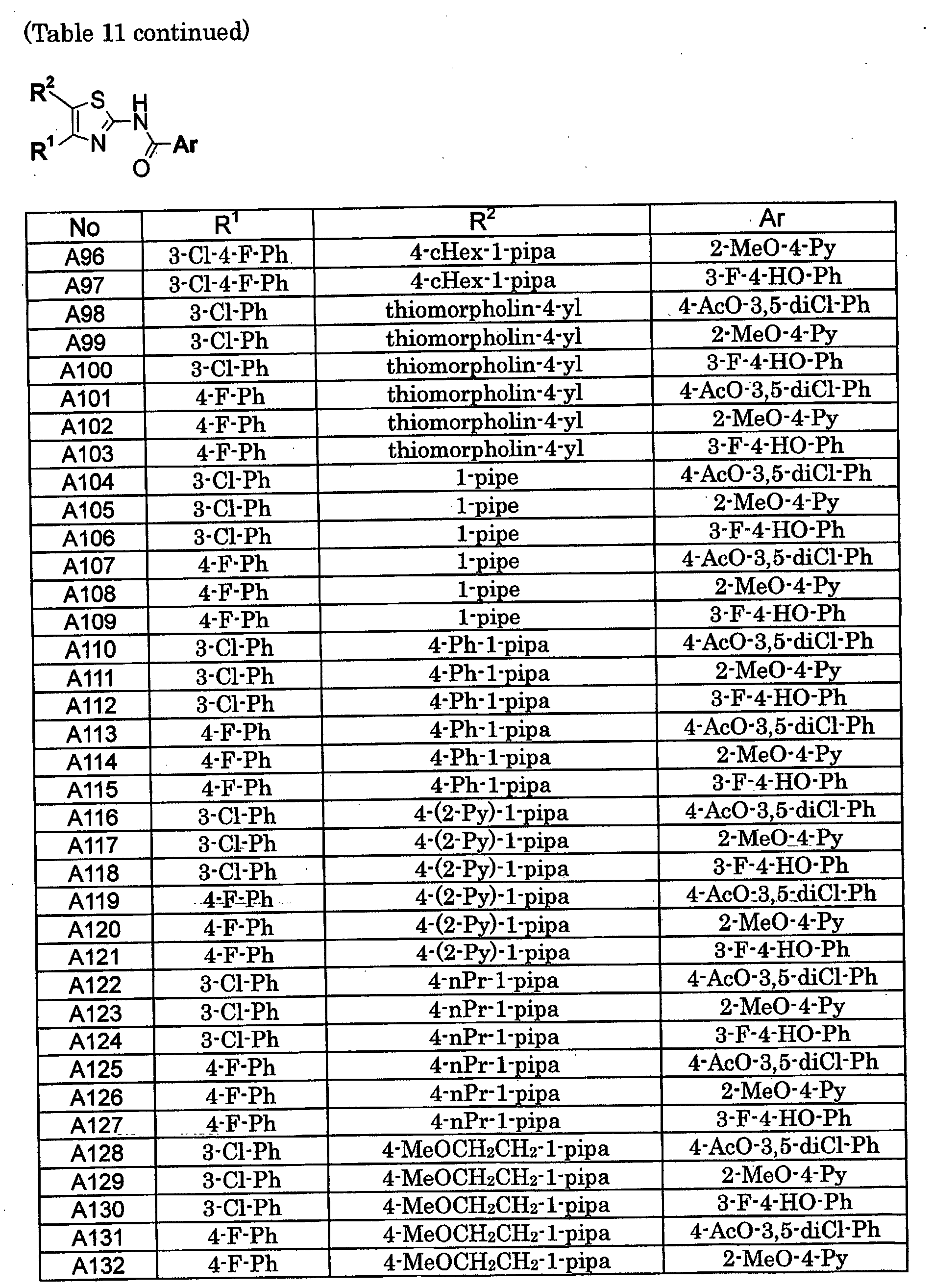

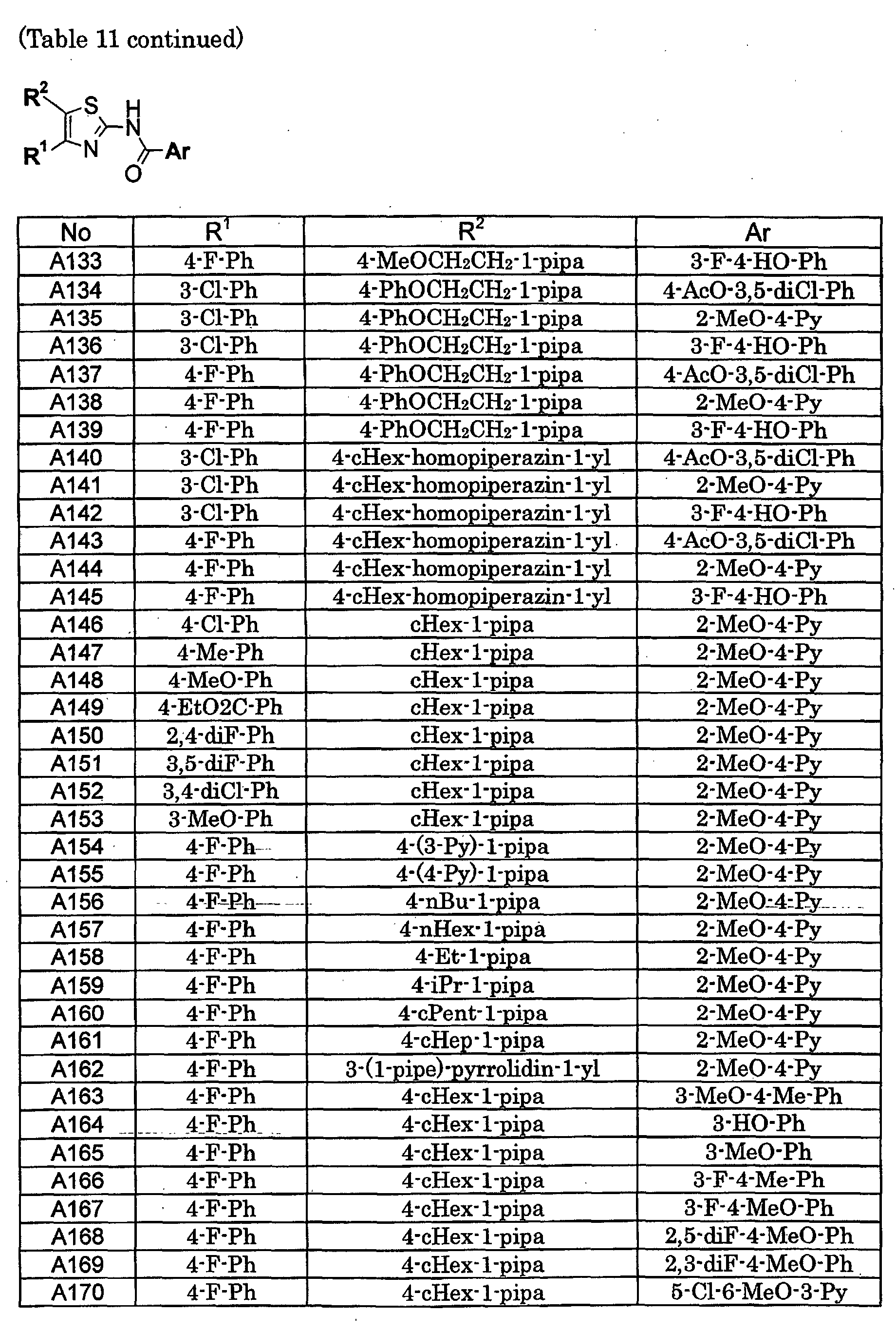

## Claims

1. A 2-acylaminothiazole derivative represented by the following general formula (I) or a pharmaceutically acceptable salt thereof 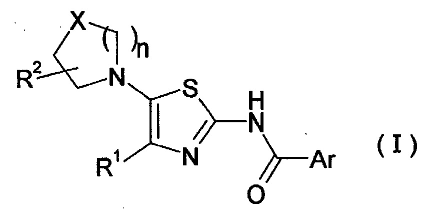 wherein:
Ar represents phenyl or pyridyl, each of which may be substituted with one or more groups selected from the group consisting of lower alkyl, -CO-lower alkyl, -COO-lower alkyl, -OH, -O-lower alkyl, -OCO-lower alkyl, and halogen;
R¹ represents aryl or pyridyl, each of which may be substituted with one or more groups selected from the group consisting of lower alkyl, -CO-lower alkyl; -COO-lower alkyl, -OH, -O-lower alkyl, -OCO-lower-alkyl, and halogen;
R² represents a group selected from the group consisting of -H, -OH, -COOH, -COO-lower alkyl, carbamoyl which may be substituted with one or two lower alkyls, amino which may be substituted with one or two lower alkyls, and cyclic amino, provided that one or more of this group may be present on the ring;
-X- represents -CH₂-, -O-, -S-, or -N(R³)-;
R³ represents optionally substituted lower alkyl, cycloalkyl, optionally substituted aryl, optionally substituted aryl-lower alkyl, optionally substituted heteroaryl, optionally substituted heteroaryl-lower alkyl, -CO-lower alkyl, -COO-lower alkyl, or carbamoyl which may be substituted one or two lower alkyls; and
n represents an integer of from 1 to 3.

2. The compound or its pharmaceutically acceptable salt according to claim 1, wherein X represents -N(R³)-, and n is 2 or 3.

3. The compound or its pharmaceutically acceptable salt according to claim 2, wherein Ar represents phenyl or pyridyl, each of which may be substituted with one or more groups selected from the group consisting of -OH, -O-lower alkyl, and -OCO-lower alkyl.

4. The compound or its pharmaceutically acceptable salt according to claim 1, wherein the compound is
3,5-dimethoxy-N-(5-morpholin-4-yl-4-phenylthiazol-2-yl)benzamide,
N-[5-(4-cyclohexylpiperazin-1-yl)-4-(4-fluorophenyl)thiazol-2-yl]-2-met hoxyisonicotinamide,
3-chloro-N-[5-(4-cyclohexylpiperazin-1-yl)-4-(4-fluorophenyl)thiazol-2-yl]-4-hydroxybenzamide,
3, 5-dimethoxy-N-(5-piperidin-1-yl-4-pyridin-4-ylthiazol-2-yl)benzamid e, or
4-{[5-(4-cyclohexylpiperazin-1-yl)-4-phenylthiazol-2-yl]carbamoyl}phe nyl acetate, or
a pharmaceutically acceptable salt thereof.

5. A pharmaceutical composition comprising, as an active ingredient, the 2-acylaminothiazole derivative or its pharmaceutically acceptable salt according to claim 1, 2, 3 or 4.

6. The pharmaceutical composition according to claim 5, as a megakaryocyte colony forming promoter.

7. The pharmaceutical composition according to claim 5, as a platelet increasing agent.

8. The pharmaceutical composition according to claim 5, as a therapeutic agent for thrombocytopenia.
